# EUROPEAN PATENT SPECIFICATION

(11) **EP 0 822 191 B1**
(45) Date of publication and mention of the grant of the patent: **19.09.2001**
(21) Application number: 97202021.8
(22) Date of filing: 02.07.1997
(51) Int. Cl.: C07D 307/92

(54) **Preparation of intermediates for norlabdane oxide**
Herstellung von Norlabdan-Oxid-Zwischenprodukten
Préparation d'intermédiaires pour l'oxyde de norlabdane

(30) Priority: 02.08.1996 EP 96305708
(43) Date of publication of application: 04.02.1998
(73) Proprietor: QUEST INTERNATIONAL B.V., 1411 GP Naarden (NL)
(72) Inventor: Davey, Paul Nicholas, Ashford, GB-TN24 OLT (GB); Payne, Laurence Sidney, Ashford, GB-TN24 OLT (GB); Tse, Chi-Lam, Ashford, GB-TN24 OLT (GB)
(74) Representative: Matthews, Heather Clare

(56) References cited:
- EP-A- 0 296 564
- EP-A- 0 752 423
- DE-A- 3 942 358
- DE-A- 4 123 767
- DE-A- 4 439 574
- US-A- 3 050 532
- US-A- 5 463 089

## Description

The present invention relates to the synthesis of the fragrance material 1,2,3a,4,5,5a,6,7,8,9,9a,9b-dodecahydro-3a,6,6,9a-tetramethylnaphto-(2,1-b)-furan, also known as (-)-norlabdane oxide.

(-)-Norlabdane oxide, of formula (1) below, is a well known fragrance material. It is the most widely used material for providing an ambergris-type odour to perfumes and is sold under various trade names, notably as Amberlyn, Ambroxan, Ambrox or Amberoxide. A number of synthetic procedures for this compound have been published. Many use naturally occurring (-)-sclareol of formula (2) below as the starting material from which the norlabdane oxide is obtained in a multistep synthesis. All processes starting from sclareol have in common that they proceed via the diol of formula (3) below as an intermediate, which has to be cyclized to give compound (1). This cyclization requires considerable care to prevent the formation of the more thermodynamically stable iso-form of (1), which has inferior olfactive properties, and the elimination of the tertiary alcohol group.

Recently US-A-5,463,089 and 5,473,085 were published which "describe the conversion of sclareol to 12-acetyl-norlabdane oxide, of formula (4) below by oxidation/rearrangement with OsO₄/NaIO₄, followed by Baeyer-Villiger oxidation with m-chloroperbenzoic acid in sodium acetate buffer to 12-acetoxy-norlabdane oxide, of formula (5) below. This acetate was thereafter reduced to (1) with LiAlH₄/BF₃.OEt₂. These US patents also provide a list of literature references describing the various procedures published for converting sclareol to norlabdane oxide.

Also various procedures are known to oxidize sclareol to sclareolide, as a first step in the final synthesis of norlabdane oxide. Chromium trioxide (SU-A-345,153), potassium permanganate (JP-A-61 033184), ruthenium tetroxide (DE-A-3942358) have all been used as well as microbial oxidation (US-A-4,798,799 and 4,970,163). In SU-A-1,409,631 a two step oxidation with ozone at low temperature, followed by treatment with alkali at high temperature, is described.

Less is known about the conversion of sclareol into sclareol oxide, of formula (6) below. L. Ruzicka et al, Helv. Chim Acta 25 (1942), 621 and D.B. Bigley et al, J. Chem. Soc. 1960 4613 describe the oxidation with KMnO₄. However, no yield was reported. B. Waegell et al, Tetrahedron Letters 1994 (35), 497 describe the conversion by Ru-catalysed oxidation but again no yield was reported In US-A-5,247,100 (equivalent to DE-A-3942358 vide supra) the conversion to sclareolide proceeds via sclareol oxide. P.F. Vlad, Khim. Prir. Soedin., 1991, (1) 43 and (4) 502 obtained sclareol oxide via ozonolysis as a mixture with other components and in a maximum yield of 54%.

Although the procedure to obtain norlabdane oxide as described in US-A-5,463,089 and 5,473,085 mentioned above provide a simplified procedure compared to those published previously, they are not well suited to use on an industrial scale. Osmium tetroxide is a very toxic chemical and therefore not suitable for large scale use. M-chloroperbenzoic acid is expensive and objectionable from an environmental point of view. Therefore, there is a need for a process to convert sclareol to norlabdane oxide in high yield, using simple, environmentally friendly and low toxicity chemicals. It is therefore an object of the present invention to provide a process for the preparation of norlabdane oxide from sclareol comprising improved process steps.

A process has now been found to produce (-)-norlabdane oxide from sclareol oxide which comprises the steps of:
I converting sclareol oxide to 12-acetylnorlabdane oxide by oxidation with an organic hydroperoxide;
II converting 12-acetylnorlabdane oxide to 12-acetoxy-norlabdane oxide by oxidation with an organic peracid.

The organic hydroperoxide used in step I is preferably a hydroperoxide derived from an aliphatic or alicyclic alcohol, particularly a tertiary alcohol. Tert-butyl hydroperoxide is very suitable. These hydroperoxides may be prepared in situ by using the corresponding alcohol as solvent or cosolvent for sclareol oxide and adding hydrogen peroxide to the solution. The reaction is preferably carried out in the presence of a catalytic amount of iodine. The reaction temperature may be chosen between 0 and 50°C, preferably between 10 and 30°C. After the reaction any excess peroxide may be removed according to usual procedures by treatment with a suitable reducing agent, such as a thiosulphate salt. The 12-acetyl-norlabdane oxide is obtained in high yield as a mixture of 12-epimers which can be used in step II without any further purification or separation.

The sclareol oxide used as the starting material in step I may be obtained by any of the methods described in the art. A preferred, novel and improved method involves the oxidation of sclareol with ozone, followed by treatment with alkaline hydrogen peroxide. This ozonolysis can be performed under usual ozonolysis conditions, using a temperature of -20 - +40°C, preferably +10 - +30°C, more preferably and for the sake of convenience at around room temperature (15-25°C). A solvent may be used which is usual for ozonolysis reactions such as a lower alcohol, a lower aliphatic acetate or methylene chloride, preferably a C1-C5 alcohol such as methanol or ethanol.

The hydrogen peroxide oxidation can be performed with dilute hydrogen peroxide, particularly 1-30% in water, in the presence of a suitable base, such as an alkaline or alkaline earth metal hydroxide, particularly NaOH or KOH. After the reaction any excess peroxide may be removed according to usual procedures by treatment with a suitable reducing agent, such as a thiosulphate salt. Sclareol oxide is obtained in high yield and can be used in step I without further purification.

The reaction with the organic peracid (step II) is carried out in a solvent which is inert to the reagent. Aliphatic or alicyclic ethers are particularly suitable, such as tert-butyl methyl ether. Although various organic peracids are suitable for the reaction, a lower (e.g. C1-C5) aliphatic peracid is preferred. Peracetic acid is particularly suitable. As usual in Baeyer-Villiger oxidations, an anhydrous buffer salt should be present, such as sodium acetate, or preferably sodium formate. The reaction can be carried out between 0 and 30°C, preferably, and for the sake of convenience at around room temperature (15-25°C). After the reaction any excess peracid may be removed according to usual procedures by treatment with a suitable reducing agent, such as a thiosulphate salt. The 12-acetoxy-norlabdane oxide is obtained in high yield and again as a mixture of epimers.

Step I and II may be carried out succesively without purification of the product of step I. They may even be carried out in one pot without isolation of the acetyl-norlabdane oxide. This also holds for the ozone oxidation of sclareol to sclareol oxide and step I. Even the total reaction sequence from sclareol to 12-acetoxy-norlabdane oxide may be carried out without purification of the intermediates, and even in one pot without isolation of the intermediates.

The 12-acetoxy-norlabdane oxide can be converted to (-)-norlabdane oxide by complex metal hydride reduction, particularly with LiAlH₄/BF₃-etherate as described in US-A-5,463,089 and 5,473,085.

A novel and very convenient process uses NaBH₄ in the presence a transition metal salt, particularly a transition metal halogenide such as ZnCl₂, FeCl₃, or CuBr. Sometimes this gives rise to a mixture of norlabdane oxide and diol (3). After separation of the mixture, the diol may be cyclized as described in the prior art.

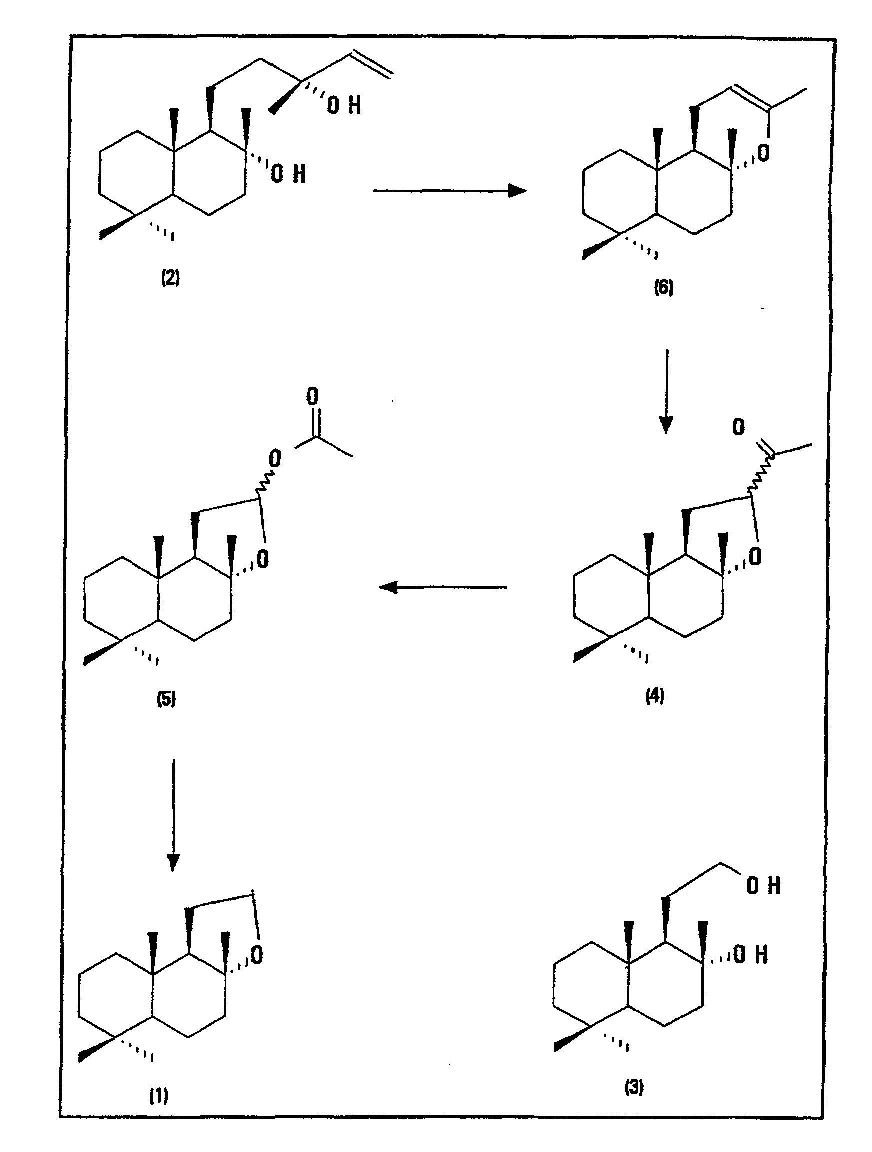

The invention is further illustrated by the following nonlimiting examples:

### Preparation of sclareol oxide

40.6g of sclareol (0.132 mol) in 300ml methanol was ozonolysed at 20-25°C using a Gebr. Herrmann LAB-50-1 lab ozoniser operating at 130V, with flow rate at 61/hr, under which conditions 0.05 mol/hr of ozone was generated. The reaction was complete in 3 hrs whereafter the reaction mixture was poured into a mixture of 19g KOH and 38ml H₂O₂ in 500ml of water and stirred for 30 min. The suspension was extracted three times with 100ml toluene. the organic layer was washed with 100ml 0.1 mol sodium thiosulphate solution and dried over Mg sulphate. Thereafter toluene was removed at 55°C under reduced pressure. 33.6g (97.4% yield) of pure sclareol oxide was obtained.

### Preparation of 12-acetyl-norlabdane oxide

To a solution of 5.5g (21 mmol) of sclareol oxide obtained above in 10ml tert-butanol was added 5ml of a 30% aqueous hydrogen peroxide solution (44.1 mmol H₂O₂) and the solution was stirred for 10 min while a white emulsion formed. To this 0.34 (1.33 mmol) of iodine was added and the mixture was stirred at room temperature for 2 hours during which it turned to orange brown due to slow dissolution of the iodine. Then the solution was warmed up to 75°C and the colour changed to deep brown, whereafter it was cooled down slowly. The reaction was shown to be completed in 2 hours by TLC and NMR. Then 25ml of saturated sodium thiosulphate was added to the reaction mixture and stirred for 30 min, during which the solution became colourless. The reaction mixture was extracted three times with 10ml tert-butyl methyl ether. The combined organic layers were washed with another 20ml of saturated sodium thiosulphate solution and dried over Mg sulphate. The solvent was evaporated and 12-acetyl-norlabdane oxide was obtained in 92% yield as a 1:5 mixture of both 12-epimers.

### Preparation of 12-acetoxy-norlabdane oxide

To a solution of 0.63g (2.25 mmol) of the epimer mixture of 12-acetoxy-norlabdane oxide obtained above in 30ml tert-butyl methyl ether was added 0.408g (6 mmol) of anhydrous sodium formate and the mixture stirred for 30 min. to obtain a homogeneous suspension. 0.6ml of a 35.5% aqueous peracetic acid solution (2.8 mmol) was added and the suspension stirred at 25-30°C for 20 hours. Thereafter 30ml of saturated sodium thiosulphate solution was added and the mixture stirred for 20 minutes. The organic layer was separated and the aqueous layer extracted twice with 30ml tert-butyl methyl ether. The combined organic layers were washed again with saturated sodium thiosulphate solution and twice with saturated sodium bicarbonate solution. The organic layer was dried over Mg sulphate and the solvent evaporated to afford 0.618g of crude 12-acetoxy-norlabdane oxide. This was purified by column chromatography using hexane and diethyl ether as eluents. 0.606g (91.5%) of the pure product was obtained as a 1:5 epimeric mixture.

### Preparation of norlabdane oxide

### Process A

To a solution of 0.532g (1.81 mmol) of the epimeric acetate mixture obtained above in 10ml diglyme 0.147g (3.88 mmol) sodium borohydride and 0.15g (1.1 mmol) of zinc chloride were added while stirring. The mixture was warmed slowly to 80°C and stirred at that temperature for 4 hours. The reaction was shown to be completed by NMR and the reaction mixture was cooled down to room temperature. Then 2ml of acetone were added to the reaction mixture and stirred for 30 minutes, whereafter the reaction mixture was poured into 20ml saturated sodium bicarbonate solution. The mixture was extracted three times with 20ml cyclohexane. The combined organic layers were washed with brine and dried over magnesium sulphate whereafter the solvent was evaporated. The crude product obtained was separated using column chromatography with hexane and diethyl ether as the eluents. 0.174g (40.8%) of norlabdane oxide was obtained and 0.251 (54%) of the diol (6).

### Process B

To a solution of the epimeric acetate mixture (5) (7.22g, 0.0246mol) in 70 ml THF, sodium borohydride (1.797g, 0.0475mol) was added, the solution was warmed up and gas evolved as the hydride was added. The suspension was stirred for ten minutes and it was cooled down with an ice bath. The anhydrous ferric chloride (3.373g, 0.02 mol) was added very slowly, gas evolved and the suspension turned black. The suspension turned rusty brown in 5 hours and according to TLC, the reaction was completed. The reaction mixture was then poured slowly with cooling into 20 ml acetone and stirred for 20 minutes. The mixture was poured into 50ml brine and 50ml cyclohexane was added. The organic layer was separated and the aqueous layer was washed with more cyclohexane (2x50 ml). The combined organic layer was dried, filtered and the solvent was stripped. 5.336g crude product was isolated. From quantitative GC analysis the yield of norlabdane oxide was 82% and of the diol 5%.

### Process C

To a solution of the epimeric acetate mixture (5) (12.02g, 0.041mol) in 50 ml THF, cooled in an ice bath, sodium borohydride (2.99g, 0.081mol) was added slowly in portions. The solution warmed up and gas evolved as the hydride was added. The suspension was stirred for 10 minutes. Copper(I) Bromide (5.86g, 0.041mol) was added very slowly, gas evolved and the suspension turned black. The reaction mixture was slowly warmed to room temperature and stirred for 1 day. The mixture was then poured slowly and with stirring into 50ml brine cooled in an ice bath. Celite (3g) was added and stirred until no more gas evolved, whereafter the mixture was filtered. The filtered black solid was washed four times with 50ml cyclohexane. The combined organic layers were washed twice with 50ml saturated ammonium chloride solution and once with brine (50ml). Thereafter it was dried. The solvent was evaporated and 8.99g crude product was obtained. According to quantitative GC analysis 86.6% yield of norlabdane oxide was obtained

## Claims

1. Process for preparing (-)-norlabdane oxide from sclareol oxide which comprises the steps of:
I converting sclareol oxide to 12-acetylnorlabdane oxide by oxidation with an organic hydroperoxide;
II converting 12-acetylnorlabdane oxide to 12-acetoxy-norlabdane oxide by oxidation with an organic peracid.

2. Process according to claim 1, wherein the hydroperoxide is derived from an aliphatic or alicyclic alcohol.

3. Process according to claim 2, wherein the hydroperoxide is derived from a tertiary alcohol.

4. Process according to claim 3, wherein the tertiary hydroperoxide is tert-butyl hydroperoxide.

5. Process according to any one of claims 2-4, wherein the hydroperoxide is prepared in situ from the corresponding alcohol and hydrogen peroxide.

6. Process according to any one of claims 1-5, wherein the organic peracid is an aliphatic C1-C5 peracid.

7. Process according to claim 6, wherein the organic peracid is peracetic acid.

8. Process according to any one of claims 1-7, wherein the sclareol oxide is prepared by ozonolysis of sclareol followed by treatment with alkaline hydrogen peroxide.

9. Process according to any one of claims 1-8, wherein the oxidation of sclareol and step I are carried out in one pot without isolation of the sclareol oxide.

10. Process according to any one of claims 1-9, wherein the oxidation with peracetic acid is carried out in the presence of sodium formate.

11. Process according to any one of claims 1-10, wherein steps I and II are carried out in one pot without isolation of the 12-acetyl-norlabdane oxide.

12. Process according to any one of claims 1-11, wherein 12-acetoxy-norlabdane oxide is converted to norlabdane oxide by reduction with NaBH₄ in the presence of a transition metal salt.

13. Process according to claim 12, wherein the transition metal salt is a halogenide.

14. Process according to claim 13, wherein the transition metal halogenide is chosen from ZnCl₂, FeCl₃ or CuBr.

## Patentansprüche

1. Verfahren zur Herstellung von (-)-Norlabdanoxid aus Sclareoloxid, umfassend die Schritte:
I Überführen von Sclareoloxid in 12-Acetylnorlabdanoxid durch Oxidation mit einem organischen Hydroperoxid;
II Überführen von 12-Acetylnorlabdanoxid in 12-Acetoxynorlabdanoxid durch Oxidation mit einer organischen Persäure.

2. Verfahren gemäß Anspruch 1, wobei das Hydroperoxid von einem aliphatischen oder alizyklischen Alkohol abgeleitet ist.

3. Verfahren gemäß Anspruch 2, wobei das Hydroperoxid aus einem tertiären Alkohol abgeleitet ist.

4. Verfahren gemäß Anspruch 3, wobei das tertiäre Hydroperoxid tert-Butylhydroperoxid ist.

5. Verfahren gemäß mindestens einem der Ansprüche 2 bis 4, wobei das Hydroperoxid in situ aus dem entsprechenden Alkohol und Wasserstoffperoxid hergestellt ist.

6. Verfahren gemäß mindestens einem der Ansprüche 1 bis 5, wobei die organische Persäure eine aliphatische C1-C5-Persäure ist.

7. Verfahren gemäß Anspruch 6, wobei die organische Persäure Peressigsäure ist.

8. Verfahren gemäß mindestens einem der Ansprüche 1 bis 7, wobei das Sclareoloxid durch Ozonolyse von Sclareol, gefolgt durch Behandlung mit alkalischem Wasserstoffperoxid hergestellt wird.

9. Verfahren gemäß mindestens einem der Ansprüche 1 bis 8, wobei die Oxidation von Sclareol und Schritt I in einem Gefäß ohne Isolierung des Sclareoloxids durchgeführt werden.

10. Verfahren gemäß mindestens einem der Ansprüche 1 bis 9, wobei die Oxidation mit Peressigsäure in Gegenwart von Natriumformiat ausgeführt wird.

11. Verfahren gemäß mindestens einem der Ansprüche 1 bis 10, wobei die Schritte I und II in einem Gefäß ohne Isolierung des 12-Acetylnorlabdanoxids durchgeführt werden.

12. Verfahren gemäß mindestens einem der Ansprüche 1 bis 11, wobei 12-Acetoxynorlabdanoxid durch Reduktion mit NaBH₄ in Gegenwart eines Übergangsmetallsalzes in Norlabdanoxid überführt wird.

13. Verfahren gemäß Anspruch 12, wobei das Übergangsmetallsalz ein Halogenid ist.

14. Verfahren gemäß Anspruch 13, wobei das Übergangsmetallhalogenid aus ZnCl₂, FeCl₃ oder CuBr ausgewählt ist.

## Revendications

1. Procédé de préparation de l'oxyde de (-)-norlabdane à partir de l'oxyde de sclaréol qui comprend les étapes consistant à :
I. convertir l'oxyde de sclaréol en oxyde de 12-acétylnorlabdane par oxydation avec un hydroperoxyde organique ;
II. convertir l'oxyde de 12-acétylnorlabdane en oxyde de 12-acétoxynorlabdane par oxydation avec un peracide organique.

2. Procédé selon la revendication 1, dans lequel l'hydroperoxyde provient d'un alcool aliphatique ou alicyclique.

3. Procédé selon la revendication 2, dans lequel l'hydroperoxyde provient d'un alcool tertiaire.

4. Procédé selon la revendication 3, dans lequel l'hydroperoxyde tertiaire est l'hydroperoxyde de tert-butyle.

5. Procédé selon l'une quelconque des revendications 2 à 4, dans lequel l'hydroperoxyde est préparé in situ à partir de l'alcool correspondant et du peroxyde d'hydrogène.

6. Procédé selon l'une quelconque des revendications 1 à 5, dans lequel le peracide organique est un peracide aliphatique en C₁ - C₅.

7. Procédé selon la revendication 6, dans lequel le peracide organique est l'acide peracétique.

8. Procédé selon l'une quelconque des revendications 1 à 7, dans lequel l'oxyde de sclaréol est préparé par ozonolyse du sclaréol suivie du traitement par un peroxyde d'hydrogène alcalin.

9. Procédé selon l'une quelconque des revendications 1 à 8, dans lequel l'oxydation du sclaréol et l'étape Is'effectuent dans un récipient sans isolement de l'oxyde de sclaréol.

10. Procédé selon l'une quelconque des revendications 1 à 9, dans lequel l'oxydation avec l'acide peracétique s'effectue en présence de formiate de sodium.

11. Procédé selon l'une quelconque des revendications 1 à 10, dans lequel les étapes I et II s'effectuent dans un récipient sans isolement de l'oxyde de 12-acétyl-norlabdane.

12. Procédé selon l'une quelconque des revendications 1 à 11, dans lequel l'oxyde de 12-acétoxynorlabdane est transformé en oxyde de norlabdane par réduction avec NaBH₄, en présence d'un sel de métal de transition.

13. Procédé selon la revendication 12, dans lequel le sel de métal de transition est un halogénure.

14. Procédé selon la revendication 13, dans lequel l'halogénure de métal de transition est choisi parmi ZnCl₂, FeCl₃ ou CuBr.
